Europäisches Patentamt

European Patent Office

Office européen des brevets

(19)

(11) **EP 1 068 510 B1**

(12) **FASCICULE DE BREVET EUROPEEN**

(45) Date de publication et mention
de la délivrance du brevet:
**12.11.2003  Bulletin 2003/46**

(21) Numéro de dépôt: **99909061.6**

(22) Date de dépôt: **19.03.1999**

(51) Int Cl.⁷: **G01N 21/35**, G01J 3/26

(86) Numéro de dépôt international:
**PCT/FR99/00658**

(87) Numéro de publication internationale:
**WO 99/049298 (30.09.1999 Gazette 1999/39)**

(54) **PROCEDE D'ETALONNAGE EN LONGUEUR D'ONDE D'UN DISPOSITIF DE FILTRAGE D'UN RAYONNEMENT ELECTROMAGNETIQUE**

VERFAHREN ZUR WELLENLÄNGENEICHUNG EINER VORRICHTUNG ZUR FILTERUNG ELEKTROMAGNETISCHER STRAHLUNG

METHOD FOR WAVELENGTH CALIBRATION OF AN ELECTROMAGNETIC RADIATION FILTERING DEVICE

(84) Etats contractants désignés:
**DE DK ES FR GB IT NL**

(30) Priorité:  **24.03.1998  FR 9803716**

(43) Date de publication de la demande:
**17.01.2001  Bulletin 2001/03**

(73) Titulaire: **SCHLUMBERGER INDUSTRIES S.A.**
**92120 Montrouge (FR)**

(72) Inventeurs:
• **DOMINGUEZ, Didier**
**N-0262 Oslo (NO)**
• **GRASDEPOT, François**
**F-92260 Fontenay aux Roses (FR)**

(74) Mandataire: **Feray, Valérie et al**
**Feray Lenne Conseil**
**44/52, Rue de la Justice**
**75020 Paris (FR)**

(56) Documents cités:
**EP-A- 0 417 884          EP-A- 0 608 049**
**WO-A-96/06345**

**Description**

L'invention est relative à un procédé d'étalonnage en longueur d'onde d'un dispositif de filtrage d'un rayonnement électromagnétique qui est inclus dans un appareil mesurant la transmission spectrale d'un milieu de propagation externe audit appareil et dans lequel milieu ledit rayonnement se propage.

On connaît des appareils tels que des analyseurs de gaz, des appareils de mesure du pouvoir calorifique de gaz ou encore par exemple des capteurs de gaz qui comprennent:

- au moins une source de rayonnement,
- un dispositif de filtrage qui présente une transmission spectrale accordable sur une gamme de longueurs d'onde dudit rayonnement en fonction de la valeur d'un paramètre physique,
- un dispositif de détection du rayonnement émis par la source, la source de rayonnement et ledit dispositif de détection étant séparés par ledit milieu de propagation.

Une des caractéristiques d'un dispositif de filtrage accordable est la relation qui existe entre la valeur V du paramètre physique appliqué audit dispositif et la longueur d'onde centrale $\lambda_{max}$ correspondant au maximum de la transmittance du dispositif de filtrage.

La relation $\lambda_{max}(V)$ peut être déterminée, par exemple, au moyen d'un spectromètre à transformée de Fourier, en mesurant la transmittance du dispositif de filtrage pour différentes valeurs V du paramètre physique appliqué audit dispositif et en identifiant les valeurs correspondantes de la longueur d'onde centrale pour lesquelles la transmittance du dispositif de filtrage est maximale.

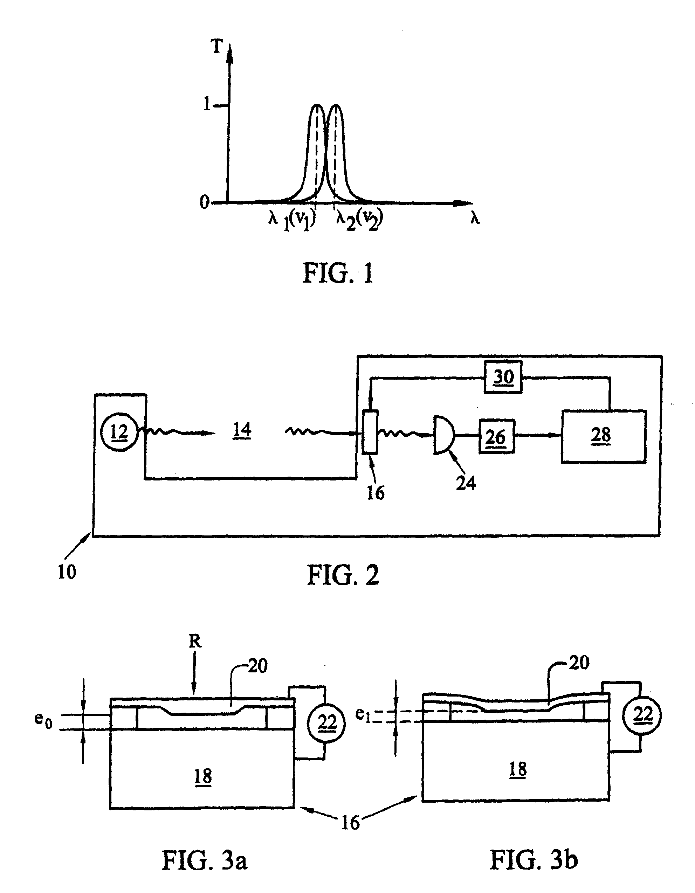

La figure 1 représente la transmission spectrale T en longueur d'onde d'un dispositif de filtrage accordé sur plusieurs longueurs d'onde centrales obtenues pour des valeurs V1 ,V2 du paramètre physique.

Cette opération d'étalonnage en longueur d'onde est généralement réalisée en laboratoire et l'étalonnage dépend alors des caractéristiques internes du spectromètre.

Ensuite, l'appareil est installé sur le terrain.

Au cours de l'utilisation de l'appareil et donc du dispositif de filtrage, il a été constaté que la relation $\lambda_{max}(V)$ entre la longueur d'onde centrale pour laquelle la transmittance du dispositif de filtrage est maximale et la valeur de contrôle V du dispositif de filtrage pouvait changer.

Un tel changement peut s'expliquer par exemple par le fait que le dispositif de filtrage est soumis au cours de son utilisation à une température différente de celle qui régnait lors de son étalonnage.

Un tel changement peut également provenir d'un vieillissement du ou des matériaux constituant le dispositif de filtrage.

Après avoir effectué cette constatation, il ne reste plus qu'à extraire l'appareil de son site d'installation, à procéder à un nouvel étalonnage du dispositif de filtrage en laboratoire, comme décrit précédemment, et à réinstaller sur site l'appareil avec le dispositif de filtrage réétalonné.

Il serait par conséquent intéressant de trouver un procédé d'étalonnage en longueur d'onde qui résolve au moins un des deux problèmes suivants : effectuer un étalonnage en laboratoire sans avoir à recourir à un spectromètre à transformée de Fourier ou effectuer l'étalonnage sans avoir à retirer l'appareil du site d'installation.

La présente invention propose à cet effet un procédé d'étalonnage en longueur d'onde d'un dispositif de filtrage d'un rayonnement électromagnétique qui est inclus dans un appareil mesurant la transmission spectrale d'un milieu de propagation externe audit appareil et dans lequel milieu ledit rayonnement se propage, ledit dispositif de filtrage présentant une transmission spectrale accordable sur une gamme de longueurs d'onde dudit rayonnement en fonction de la valeur d'un paramètre physique, caractérisé en ce que ledit procédé consiste à:

- sélectionner au moins une raie gazeuse d'absorption qui soit toujours présente à l'état naturel dans le milieu de propagation et dont la longueur d'onde correspondante soit incluse dans ladite gamme de longueurs d'onde d'accordabilité du dispositif de filtrage,
- et étalonner le dispositif de filtrage par rapport à ladite au moins une raie gazeuse d'absorption qui sert de référence naturelle.

Ce procédé est particulièrement simple à mettre en oeuvre puisqu'il ne nécessite pas de modifier l'appareil dans lequel est inclus le dispositif de filtrage en y incluant artificiellement par exemple une cellule contenant un gaz de référence.

Préférentiellement, ladite au moins une raie gazeuse d'absorption est d'une largeur spectrale inférieure ou égale à celle du dispositif de filtrage et est suffisamment intense pour ne pas être masquée par d'autres raies gazeuses.

Un tel procédé peut donc être avantageusement utilisé pour l'étalonnage du dispositif de filtrage lorsque l'appareil dans lequel il est inclus est installé sur son site d'utilisation.

Ainsi, grâce à ce procédé il n'est plus nécessaire de ramener l'appareil en laboratoire pour procéder à son étalonnage puisque la raie gazeuse d'absorption servant de référence est présente naturellement dans le milieu de propagation.

Le milieu de propagation peut être par exemple l'atmosphère et l'appareil, un capteur de monoxyde de carbone utilisant la(es) raie(s) du dioxyde de carbone présente(s) dans l'atmosphère comme référence(s) naturelle(s).

De préférence, l'appareil comprend en outre :

- au moins une source du rayonnement électromagnétique et
- un dispositif de détection du rayonnement émis par la source, ladite source et ledit dispositif de détection étant séparés par le milieu de propagation.

Il est également possible qu'un volume de gaz dont on souhaite mesurer la transmission spectrale soit interposé entre la source et le dispositif de détection et que ce volume de gaz contienne des raies gazeuses d'absorption pouvant servir de références naturelles conformément au procédé selon l'invention.

Dans ce cas le volume de gaz interposé tient lieu de milieu de propagation au sens de l'invention.

Si le volume de gaz interposé n'occupe pas tout le volume entre la source et le dispositif de détection il est alors également possible de choisir entre les raies gazeuses naturelles du volume de gaz et les raies gazeuses naturelles présentes dans le volume restant non occupé entre ladite source et ledit dispositif celles que l'on souhaite utiliser.

Ce procédé peut également s'appliquer en laboratoire pour étalonner le dispositif de filtrage avant sa première mise en service sans avoir à recourir à un spectromètre à transformée de Fourier.

Plus particulièrement, le procédé selon l'invention consiste successivement à :

- faire varier le paramètre physique appliqué au dispositif de filtrage pour faire coïncider le maximum de la transmission spectrale dudit dispositif de filtrage avec la longueur d'onde de la raie gazeuse de référence,
- en déduire le(s) coefficients) de la loi qui régit l'accordabilité du dispositif de filtrage en longueur d'onde, l'allure générale de ladite loi étant préalablement connue,
- déterminer à partir de cette loi d'autres valeurs du paramètre physique correspondant chacune à une longueur d'onde sur laquelle est accordée la transmission spectrale du dispositif de filtrage lors de son utilisation.

Il est possible par exemple de sélectionner sur ladite gamme de longueurs d'onde du rayonnement électromagnétique la raie gazeuse d'absorption qui présente la plus forte intensité par rapport aux autres raies gazeuses d'absorption.

Lors de l'étalonnage du dispositif de filtrage, l'identification de cette raie gazeuse est aisée puisqu'elle correspond à l'absorption maximale sur la gamme de longueurs d'onde.

Il est également avantageux de sélectionner sur ladite gamme de longueurs d'onde du rayonnement électromagnétique deux raies gazeuses d'absorption au lieu d'une seule pour garantir une plus grande fiabilité de l'étalonnage.

Préférentiellement, le rayonnement électromagnétique est du type infra-rouge.

L'une des raies gazeuses d'absorption est par exemple celle du méthane à 1,666 microns.

Il peut être également intéressant de sélectionner la raie d'absorption du méthane à 1,791 microns, ce en fonction de la gamme de longueurs d'onde envisagées et de l'étendue d'accordabilité du dispositif de Filtrage.

Préférentiellement, le procédé consiste à appliquer au dispositif de filtrage comme paramètre physique un champ électrique sous la forme d'une tension électrique mais il pourrait également s'agir d'un champ magnétique.

Selon d'autres caractéristiques :

- le dispositif de filtrage est un interféromètre de Fabry-Perot,
- l'interféromètre de Fabry-Perot est un interféromètre court,
- l'interféromètre de Fabry-Perot est un interféromètre micro-usiné,
- l'appareil est un analyseur de gaz,
- l'appareil est un appareil de mesure du pouvoir calorifique du gaz,
- l'appareil est un capteur de gaz.

D'autres caractéristiques et avantages de l'invention apparaîtront au cours de la description qui va suivre donnée à titre d'exemple non limitatif, et faite en référence aux dessins annexés sur lesquels :

- la figure 1 a déjà été décrite,
- la figure 2 représente schématiquement les différents éléments constituant un capteur de gaz,
- les figures 3a et 3b représentent deux positions successives d'un dispositif de filtrage accordable électriquement pour deux valeurs de tension différentes,

- la figure 4 représente schématiquement les différents éléments constituant un capteur de gaz dans une application différente de celle du capteur représenté à la figure 2,
- la figure 5 donne l'allure des raies gazeuses d'absorption du méthane,
- les figures 6 et 7 fournissent respectivement l'intensité I (en $atm^{-1}.cm$) des spectres de rotation-vibration des molécules d'eau et de dioxyde de carbone en fonction du nombre d'onde ($cm^{-1}$).

Un appareil de détermination de la concentration d'un gaz, tel que par exemple un capteur de monoxyde de carbone, est représenté sur la figure 2 et désigné par la référence générale notée 10.

Cet appareil comprend une source 12 d'émission d'un rayonnement électromagnétique qui est préférentiellement un rayonnement situé dans l'infra-rouge. Ce rayonnement est émis à travers un milieu de propagation 14 extérieur à l'appareil et qui est par exemple l'atmosphère.

Toutefois, un rayonnement situé dans le visible, ou dans l'ultra-violet ou bien dans le domaine des hyperfréquences ou encore dans le domaine des rayons X pourrait également convenir.

La source de rayonnement infra-rouge 12 est par exemple une source à large bande constituée d'un filament de tungstène et émet un rayonnement dont les longueurs d'onde sont comprises entre 0,8 et 20μm.

L'appareil 10 comprend un dispositif de filtrage 16 du rayonnement qui est émis par la source 12 et qui s'est propagé dans le milieu 14.

Ce dispositif pourrait également être placé directement devant la source 12 sans que cela ne modifie le fonctionnement de l'appareil.

Le dispositif de filtrage 16 est par exemple un interféromètre de Fabry-Pérot court (l'ordre de l'interféromètre étant par exemple de 10).

Ce dispositif de filtrage peut être réalisé en silicium et fabriqué par des techniques de micro-usinage connues.

Un tel dispositif de filtrage est par exemple décrit dans les documents EP 0 608 049 et EP 0 219 359.

Comme représenté sur les figures 3a et 3b, le dispositif de filtrage 16 est constitué d'une électrode fixe 18 formant un support et d'une électrode mobile 20 séparées l'une de l'autre d'une distance déterminée $e_0$ correspondant à une position dans laquelle l'électrode mobile n'est pas déformée.

Dans cette position dite de repos le rayonnement indiqué par la flèche repérée par la lettre R sur la figure 3a est filtré pour la longueur d'onde $\lambda_0$ qui est égale à $2e_0$ (et pour les harmoniques de cette longueur d'onde).

Le dispositif de filtrage 16 présente une transmission spectrale T (représentée à la figure 1) qui est accordable sur une gamme de longueurs d'onde du rayonnement infra-rouge en fonction de la valeur d'un paramètre physique qui est par exemple un champ électromagnétique, c'est-à-dire qu'il est possible de faire coïncider le maximum de transmittance du dispositif de filtrage avec différentes longueurs d'onde incluses dans ladite gamme en faisant varier le champ électromagnétique appliqué audit dispositif de filtrage.

Le champ électromagnétique est plus précisément Un champ électrique créé par une source de tension 22.

Cependant, il pourrait également s'agir d'un champ magnétique. Un aimant, par exemple, peut être fixé sur l'électrode fixe, et une bobine peut être déposée sur l'électrode mobile (ou l'inverse). Un courant circulant dans la bobine permet de rapprocher l'électrode mobile de l'électrode fixe et donc de déplacer la longueur d'onde sur laquelle est accordée le dispositif de filtrage.

Le paramètre physique pourrait également être la température. Dans ce cas, les électrodes mobile et fixe pourraient être séparées par une cale réalisée dans un matériau à fort coefficient de dilatation thermique qui, sous l'effet d'une variation de température, induirait une variation de la distance entre les électrodes et donc une accordabilité du dispositif de filtrage sur une longueur d'onde particulière.

La source de tension 22 est reliée aux électrodes mobile et fixe et, lorqu'une tension est appliquée (Fig.3b), l'électrode mobile se déforme et se rapproche de l'électrode fixe. La distance entre les électrodes se réduit à $e_1$ ($e_1 < e_0$) et, le rayonnement est alors filtré pour la longueur d'onde $\lambda_1$ égale à $2e_1$. Ainsi, pour différentes valeurs de tension électrique le dispositif de filtrage s'accorde sur différentes longueurs d'onde.

La gamme de longueurs d'onde s'étend par exemple de 4 à 5 μm.

L'appareil 10 comprend en outre un dispositif de détection 24 du rayonnement qui est partiellement absorbé dans le milieu de propagation 14 et qui est filtré par le dispositif de filtrage 16.

Le dispositif de détection 24 est un détecteur à large bande tel que par exemple un bolomètre, une thermopile ou une photodiode.

L'énergie contenue dans le rayonnement infra-rouge et reçue par le détecteur est transformé en un signal électrique représentatif de ce rayonnement.

Le signal est ensuite amplifié, converti en signal numérique par le convertisseur 26 puis injecté dans un microprocesseur 28.

Un convertisseur numérique-analogique 30 est utilisé pour accorder la transmission spectrale du filtre 16 sur différentes longueurs d'onde.

En première approximation, on peut considérer la transmittance d'un filtre interférentiel comme une gaus-

sienne

$$T_f(V) = T_{max}(V)\exp{-\left(\frac{\lambda - \lambda_{max}(V)}{\sigma(V)}\right)^2}$$

où $V$ est la tension de commande du filtre, $\lambda_{max}$ la longueur d'onde du maximum de transmission, et $\sigma$ sa largeur.

Pour le dispositif de filtrage accordable 16, la longueur d'onde $\lambda_{max}$ du maximum de transmission varie avec la tension de commande V suivant la relation:

$$\lambda_{max}(\lambda_{max} - \lambda_{max0}) + (KV)^2 = 0 \qquad\qquad [1]$$

où

$\lambda_{max0}$ (en µm) est la longueur d'onde du maximum de transmission du dispositif de filtrage à V=0 (=$V_0$), et K (en µm/V) une constante dépendant de la construction du dispositif de filtrage.

Le dispositif de filtrage est conçu pour être accordé de 5µm à 4µm avec des tensions appliquées de 0 à 20V, ce qui correspond à une valeur de $K$ sensiblement égale à 0,10 µm/V.

L'opération d'étalonnage en longueur d'onde consistant à déterminer initialement (c'est-à-dire avant toute utilisation du dispositif de filtrage 16 et du capteur 10) la relation $\lambda_{max}$ (V) est réalisée en laboratoire au moyen d'un spectromètre à transformée de Fourier, de même que l'opération d'étalonnage en amplitude du capteur, et les données résultant de ces opérations sont mémorisées dans le microprocesseur 28. Le dispositif de filtrage est ensuite monté dans le capteur et ce dernier est installé sur le lieu de son utilisation.

Or, par exemple, lorsque la température à laquelle est soumis le dispositif de filtrage en utilisation est différente de celle régnant autour dudit dispositif de filtrage lors de son étalonnage, il peut se produire une dérive du dispositif de filtrage en longueur d'onde qui se traduit par exemple par une accordabilité du dispositif de filtrage sur les longueurs d'onde égales à 4,9 et 3,8 µm pour les tensions respectives de 0 et 20V au lieu des longueurs d'onde respectives 5 et 4µm.

Si aucune correction n'est apportée le capteur de gaz perdra de sa précision dans la détermination de la concentration de monoxyde de carbone.

L'invention prévoit d'utiliser au moins une raie d'absorption d'une substance gazeuse présente naturellement dans le volume de gaz à analyser pour étalonner en fréquence le dispositif de filtrage 16 in-situ.

Le procédé selon l'invention consiste à sélectionner sur la gamme de longueurs d'onde s'étendant de 4 à 5 µm une longueur d'onde particulière correspondant à une raie gazeuse d'absorption qui est toujours présente sur cette gamme.

Il ne faudrait effectivement pas choisir une raie d'absorption, comme par exemple celle d'un gaz interférent, qui pourrait disparaître au cours du temps ou consécutivement aux variations de divers paramètres tels que la pression ou la température.

Dans le cas présent, le choix d'une raie gazeuse de dioxyde de carbone est particulièrement judicieux car la position des raies gazeuses du $CO_2$ ne dépend ni de la pression ni de la température et ces raies sont toujours présentes dans l'atmosphère.

Pour d'autres applications (environnement différent, gamme de longueurs d'onde différentes...) il pourrait par exemple être intéressant de sélectionner une raie gazeuse d'absorption de la vapeur d'eau pour des mesures atmosphériques ou une raie gazeuse d'absorption du méthane.

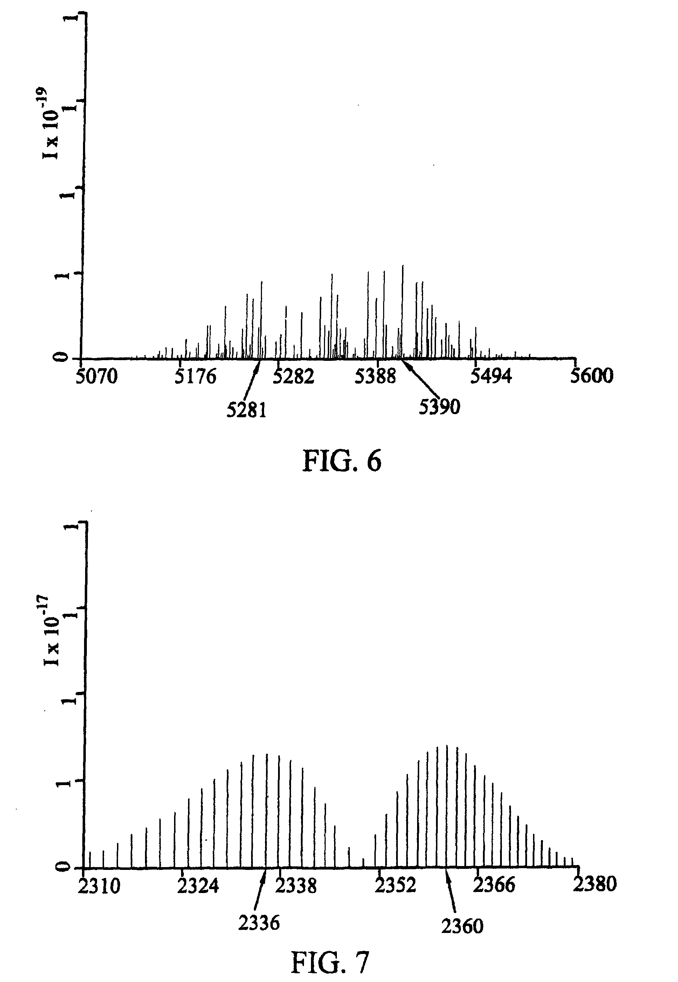

On trouve des raies d'absorption de ces gaz à de nombreuses longueurs d'onde: 1,893µm (nombre d'onde d'environ 5281cm$^{-1}$) ou 1,855 µm (nombre d'onde d'environ 5390cm$^{-1}$) pour $H_2O$ (fig. 6), 4,280µm (nombre d'onde d'environ 2336cm$^{-1}$) ou 4,237µm (nombre d'onde d'environ 2360cm$^{-1}$) pour $CO_2$ (Fig.7).

Préférentiellement, la raie d'absorption sélectionnée doit être fine, c'est-à-dire que sa largeur doit être inférieure ou égale à la largeur spectrale du dispositif de filtrage dans sa partie formant un pic centré autour de la transmission maximale afin que d'éventuels déplacements de la transmission spectrale dudit dispositif de filtrage puissent être décelés.

De préférence, cette raie d'absorption doit en outre être suffisamment intense par rapport aux autres raies gazeuses présentes sur la gamme de longueurs d'onde pour pouvoir être aisément distinguée de celles-ci.

Si la raie sélectionnée risquait d'être masquée par d'autres raies gazeuses celle-ci perdrait tout son intérêt comme référence naturelle pour l'étalonnage. La raie d'absorption du dioxyde de carbone que l'on trouve pour une longueur d'onde de 4,237 µm répond aux critères précédemment définis pour cette application et peut donc être utilisée comme référence naturelle.

**[0072]** La largeur spectrale de la raie choisie est voisine de 1nm alors que celle du dispositif de filtrage est voisine de 10nm.

**[0073]** Avant toute utilisation du dispositif de filtrage, comme précédemment expliqué, celui-ci est étalonné en longueur d'onde (relation $\lambda$ (V)) et le capteur est étalonné en amplitude avec un gaz étalon dont on connaît la composition afin de mémoriser dans le microprocesseur 28 les couples de valeurs tension / longueur d'onde vérifiant la relation [1] et qui correspondent à des points de fonctionnement du dispositif de filtrage au cours de son utilisation.

**[0074]** Ces couples mémorisés doivent comprendre le couple $V_i$ / 4,237 µm correspondant au point de fonctionnement du dispositif de filtrage lorsque la transmission spectrale de celui-ci est accordée sur la longueur d'onde 4,237 µm sous une tension électrique égale à $V_i$.

**[0075]** Il est également possible de ne mémoriser que ce couple $V_i$/4,237µm lors de l'étalonnage et de calculer et mémoriser ultérieurement les autres couples de valeurs tension/longueur d'onde correspondant à des points de fonctionnement du dispositif de filtrage au cours de son utilisation.

**[0076]** Après avoir utilisé le capteur 10 intégrant le dispositif de filtrage 16, soit après une durée prédéterminée, soit pour une raison particulière, on peut souhaiter étalonner de nouveau le dispositif de filtrage en longueur d'onde. Pour ce faire, on applique par l'intermédiaire du microprocesseur 28 une série de tensions au dispositif de filtrage 16 ce qui a pour effet d'accorder la transmission spectrale de celui-ci sur différentes longueurs d'onde de la gamme [4 ; 5µm]. On recueille pour chaque tension le signal correspondant en sortie du détecteur 24.

**[0077]** Connaissant la tension initiale $V_i$ pour laquelle la transmission spectrale du dispositif de filtrage était accordée sur 4,237 µm, le microprocesseur 28 identifie parmi les signaux numérisés celui qui a la plus faible intensité pour une position du dispositif de filtrage correspondant à une tension voisine de $V_{i.}$

**[0078]** La valeur $V_f$ de la tension pour laquelle ce signal est obtenu est mémorisée en association avec la longueur d'onde de 4,237 µm et la dérive du dispositif de filtrage est donnée par la différence $\Delta V = | V_f - V_i |$

**[0079]** Connaissant cette dérive et connaissant les longueurs d'onde $\lambda_{max}$ sur lesquelles la transmission spectrale du dispositif de filtrage doit être accordée, les nouvelles valeurs V' de la tension pour lesquelles cette accordabilité peut être obtenue sont déduites de la relation $V' = V + \Delta V$.

**[0080]** Ce procédé est avantageux car il permet un réétalonnage de la relation $\lambda_{max}$ (V) du dispositif de filtrage en au moins un point où une vérification de sa stabilité en longueur d'onde sans démontage du dispositif de filtrage 16 ni du capteur 10.

**[0081]** Le réétalonnage en plusieurs points permet de pouvoir prendre en compte des relations $\lambda$(V) plus compliquées que celle présentée plus haut.

**[0082]** En outre, ce procédé est indépendant de la température et de la pression puisque la raie de référence n'est pas sensible à ces grandeurs.

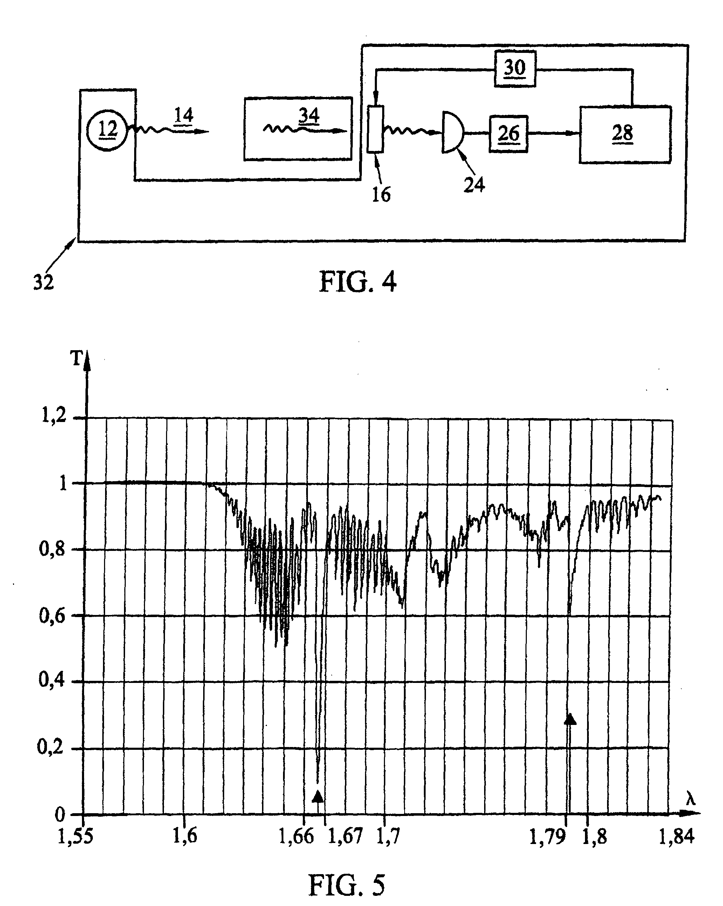

**[0083]** Dans une autre application illustrée par l'appareil 32 de la figure 4, l'appareil de la figure 2 conserve la même structure que celle décrite précédemment mais une cellule 34 renfermant un échantillon de gaz à analyser est placée dans le milieu de propagation 14 sur le parcours du rayonnement infra-rouge émis par la source 12.

**[0084]** Les éléments identiques à ceux de la figure 2 conservent les mêmes références.

**[0085]** Il est possible, soit de conserver comme référence(s) la(es) raie(s) gazeuse(s) d'absorption présente(s) naturellement dans le milieu de propagation (exemple : raies du $CO_2$ ou de la vapeur d'eau) auquel cas, le procédé d'étalonnage du dispositif de filtrage 16 tel que décrit précédemment s'applique de façon analogue, soit de sélectionner une ou plusieurs raies gazeuses d'absorption présentes naturellement dans le gaz de la cellule dont on souhaite mesurer la transmission spectrale et de s'en servir pour étalonner le dispositif de filtrage 16 en longueur d'onde.

**[0086]** Cette dernière solution pourrait par exemple consister à sélectionner des raies du méthane dans une cellule renfermant du gaz naturel dont on cherche à mesurer le pouvoir calorifique.

**[0087]** Selon une variante de réalisation, le procédé selon l'invention peut consister à sélectionner deux raies gazeuses d'absorption dont les longueurs d'onde sont situées sur la gamme de longueurs d'onde considérée [4 ; 5 µm] qui sont fines, intenses et toujours présentes sur cette gamme.

**[0088]** Par exemple, les deux raies peuvent être celles du $CO_2$ à 4,237 et à 4,280 µm.

**[0089]** Il est avantageux de sélectionner une deuxième raie gazeuse d'absorption et de l'utiliser pour l'étalonnage du dispositif de filtrage compte tenu du fait que le(s) matériau(x) constitutif(s) dudit dispositif de filtrage peuvent vieillir au cours du temps. Ainsi, le module de Young du matériau peut varier induisant par là même une variation de la constante K précédemment définie.

**[0090]** En utilisant les deux raies gazeuses d'absorption précédentes, les 2 paramètres, $\lambda_{max0}$ et $K$ caractérisant la relation [1], peuvent être déterminés de la façon suivante :

**[0091]** On recherche les tensions $V_1$ et $V_2$ correspondant au premier et au deuxième minimum de la transmitance du dispositif de filtrage 16 ce qui consiste, en fait, à une recherche de minimum local autour de chaque valeur de tension pour laquelle, initialement, le dispositif de filtrage était accordé sur la longueur d'onde de la raie de référence.

**[0092]** A partir des valeurs de $V_1$ et $V_2$ ainsi obtenues on peut calculer:

$$\lambda_{max0} \frac{\lambda_1^2 V_2^2 - \lambda_2^2 V_1^2}{\lambda_1 \, V_2^2 - \lambda_2 \, V_1^2}$$

et

$$K = \frac{\sqrt{\lambda_1 \left( \frac{\lambda_1^2 \, V_2^2 - \lambda_2^2 \, V_1^2}{\lambda_1 \, V_2^2 - \lambda_2 \, V_1^2} - \lambda_1 \right)}}{V_1}$$

Les valeurs de tension à appliquer au dispositif de filtrage pour obtenir les longueurs d'ondes souhaitées peuvent alors se calculer à partir de la relation [1] et des coefficients $\lambda_{max0}$ et K que l'on vient de déterminer.

Il convient de noter qu'il peut être intéressant de sélectionner plus de deux raies gazeuses d'absorption, par exemple afin d'améliorer la précision du réétalonnage.

La recherche de ces raies peut être faite en repérant l'ordre dans lequel elles apparaissent lors du premier étalonnage et en les indexant.

Le procédé selon l'invention peut également être appliqué à un dispositif de filtrage inclus dans un analyseur d'un mélange de gaz ou dans un appareil de mesure du pouvoir calorifique d'un gaz, tel que par exemple le gaz naturel.

Ce dernier comporte les mêmes éléments 12 à 28 que ceux représentés sur les figures 2, 3a et 3b.

La gamme de longueurs d'onde sur laquelle la transmission spectrale du dispositif de filtrage 16 peut s'accorder s'étend par exemple de 1,50 à 1,85 µm.

Dans le gaz naturel, le méthane est un constituant majoritaire toujours présent il est donc particulièrement intéressant de sélectionner une raie d'absorption du méthane comme référence naturelle pour procéder à l'étalonnage en fréquence du dispositif de filtrage 16 in-situ.

La raie correspondant à 1,666 µm est la plus intense de toutes les raies d'absorption sur la gamme de longueurs d'onde envisagées et elle est suffisamment fine ($\approx$1nm) par rapport à la largeur spectrale du dispositif de filtrage 16 ($\approx$10nm).

Le procédé d'étalonnage selon l'invention est mis en oeuvre de la même manière qu'indiquée précédemment pour le capteur de CO.

Etant donné que la raie d'absorption à 1,666 µm est la plus intense de toutes les raies sur la gamme [1,50 ; 1,85 µm], il est aisé de détecter le signal minimum en sortie du détecteur 24.

Comme indiqué par les flèches de la figure 5 qui représente la transmittance T en fonction de la longueur d'onde $\lambda$, deux raies d'absorption du méthane peuvent être sélectionnées sur la gamme [1,50 ; 1,85 µm], la raie à 1,666 et celle à 1,731 µm.

On obtient ainsi les avantages évoqués précédemment pour le capteur de CO.

A titre d'exemple, le gaz naturel possède la composition suivante :

| | |
|---|---|
| Méthane | 89,5 % |
| Ethane | 5 % |
| Propane | 1 % |
| Butane | 0,6 % |
| Pentane | 0,3 % |
| Gaz neutres | 3,6 % |

Plusieurs longueurs d'onde $\lambda_1$ à $\lambda_5$ vont être utilisées pour déterminer la contribution des différents constituants du gaz naturel cités ci-dessus à l'exception des gaz neutres qui n'apportent aucune contribution au pouvoir calorifique.

Ces longueurs d'onde sont telles qu'à chacune d'elles correspond la contribution de plusieurs constituants combustibles.

En appliquant une tension V de valeur déterminée par exemple égale à 20V au dispositif de filtrage 16 celui-ci s'accorde sur la longueur d'onde $\lambda_1$ et le détecteur 24 fournit un signal électrique correspondant à $S_1$ (V) :

$$S1(V) = \int_\lambda E\,(\lambda)\,\theta gaz\,(\lambda,\,xi)\,\theta f\,(\lambda,\,V)\,Sd\,(\lambda)\,d\lambda$$

où E $(\lambda)$ désigne l'intensité lumineuse émise par la source 12,

$$\theta gaz\,(\lambda,\,x_i) = \exp\,(-\,L\,\sum_i \alpha_i\,(\lambda).\,xi)$$

désigne la réponse spectrale due à tous les constituants combustibles gazeux présents à cette longueur d'onde,
L désignant la longueur du trajet optique dans le gaz,
$x_i$ représentant le nombre de moles du constituant combustible i par unité de volume à la pression P et à la température T,
$\alpha_i$ désignant le coefficient d'absorption du constituant combustible i, et dépendant de la longueur d'onde, de la pression et de la température,
$\theta f$ $(\lambda,\,V)$ représentant la transmission optique due au dispositif de filtrage 16 et Sd représentant la réponse spectrale du détecteur.

En accordant le dispositif de filtrage 16 sur les différentes longueurs d'onde $\lambda_1$ à $\lambda_5$ pour différentes valeurs de tension $V_1$ à $V_5$ on mesure les valeurs $S_1(V_1)$ à $S_5(V_5)$. L'absorbance A se définit comme suit $A(V) = Ln(1/S(V))$, où Ln désigne la fonction logarithme népérien, et l'on obtient le système suivant de cinq équations :

$$A_1(V_1) = a_{11}x_1 + a_{21}x_2 + ..... + a_{51}x_5$$

$$A_2(V_2) = a_{12}x_1 + a_{22}x_2 + ..... + a_{52}x_5$$

$$........................................$$

$$A_5(V_5) = a_{15}x_1 + a_{25}x_2 + ..... + a_{55}x_5$$

où les termes aij dépendent du constituant i et de l'appareil 10.

Avant de mettre en oeuvre l'invention sur un gaz naturel de composition non connue on procède à une étape d'étalonnage préalable en laboratoire en injectant dans l'appareil 10 plusieurs gaz avec des constituants de nombres de moles par unité de volume xi connues à T et P données.

L'étape d'étalonnage en longueur d'onde du dispositif de filtrage effectuée en laboratoire est habituellement réalisée au moyen d'un spectromètre à transformée de Fourier.

Le procédé selon l'invention permet d'effectuer cet étalonnage sans avoir à recourir à un spectromètre, en utilisant uniquement par exemple les deux raies du méthane à 1,666 et à 1,791 μm.

Pour ce faire on injecte un premier mélange connu de gaz dans l'appareil 10 et l'on fait varier la tension V appliquée au dispositif de filtrage 16 pour faire coïncider le maximum de transmission dudit dispositif de filtrage avec les longueurs d'onde des raies de référence du méthane précitées.

Dès que les valeurs $V_a$ et $V_b$ de la tension pour lesquelles la transmission spectrale du dispositif de filtrage est accordée sur les longueurs d'onde 1,666 et 1,791 μm sont obtenues, les paramètres K et $\lambda_{max0}(V)$ sont déterminés de la manière déjà indiquée plus haut et la relation $\lambda_{max}(V)$ est donc parfaitement connue.

Connaissant cette relation $\lambda_{max}(V)$ qui s'écrit sous la forme

$$\lambda_{max}(V)\,(\lambda_{max}(V) - \lambda_{max0}) + (KV)^2 = 0$$

ainsi que les longueurs d'onde $\lambda_1(i = 1, 5)$, on en déduit facilement les tensions $V_i$ (i = 1, ..., 5) correspondant respectivement aux positions du dispositif de filtrage 16 pour lesquelles le maximum de transmission dudit dispositif de filtrage coïncide avec les longueurs d'onde $\lambda_i$.

Les valeurs K, $\lambda_{max0}$ et les couples $V_a$ / 1, 666 μm, $V_b$ / 1, 791 μm et $V_i$ / $\lambda_i$ sont mémorisés dans le microprocesseur 28 de la figure 4.

Les tensions $V_i$ (i = 1, ...,5) ainsi obtenues sont successivement appliquées au dispositif de filtrage pour que sa transmission spectrale s'accorde sur les longueurs d'onde $\lambda_i$ (i = 1,...,5) et pour chaque couple $V_i$ /$\lambda_1$, le détecteur

fournit une valeur $S_{i1}(V_i)$.

Ainsi, on obtient un système de cinq équations :

$$A_{11}(V_1) = a_{11}x_1 + ... + a_{51}x_5$$

$$...$$

$$A_{51}(V_5) = a_{15}x_1 + ... + a_{55}x_5$$

où les $x_i$ (i = 1, ..., 5) sont connues et où les termes $a_{ij}$ sont les inconnues.

En injectant dans l'appareil 10 quatre autres mélanges de gaz connus on obtient ainsi vingt équations supplémentaires avec les mêmes termes $a_{ij}$ que précédemment.

Ceci permet alors de calculer par une méthode mathématique connue, telle que par exemple une méthode de résolution d'équations linéaires, les coefficients $a_{ij}$ qui sont définis comme suit :

$$[A_j] = [a_{ij}][x_i]$$
$$k = 1, ..., 5 \qquad k = 1, ..., 5$$

où les indices k identifient le mélange de gaz connu concerné.

Il convient de noter, à titre de variante, que l'on peut effectuer une mesure de référence en choisissant une longueur d'onde à laquelle ne correspond la contribution d'aucun constituant du mélange de gaz et la tension correspondante se déduit de la relation $\lambda_{max}(V)$ citée plus haut

On applique cette tension au dispositif de filtrage et l'on recueille en sortie de détecteur la valeur $S_{ref}$, puis on fait le rapport de chacune des valeurs $S_{ij}(V_i)$ avec cette valeur $S_{ref}$. Ce rapport $S_{ij}(V_i) / S_{ref}$ est ensuite utilisé à la place de la valeur $S_{ij}(V_i)$ dans l'exposé précédent et ceci permet d'éliminer les dérives de l'appareil 10.

En inversant par une méthode mathématique d'inversion classique la matrice $[a_{ij}]$, on se ramène à un système d'équations

$$[x_i] = [a_{ij}]^{-1}[A_j] \quad = \quad [b_{ij}][A_j]$$
$$x = 1, ..., 5 \quad i = 1, ..., 5 \quad i = 1, ..., 5 \qquad i = 1, ..., 5 \quad i = 1, ..., 5$$
$$j = 1, ..., 5 \qquad\qquad j = 1, ..., 5$$

Ainsi, les valeurs $x_i$ s'écrivent

$$x_i = \sum_{j = 1, ..., 5} b_{ij} A_j(V),$$

Il suffit de mémoriser dans la mémoire du microprocesseur 28 les données $b_{ij}$ calculées lors de l'étalonnage, et lorsque l'on a affaire à un gaz naturel de composition et donc de pouvoir calorifique non connus, les différentes valeurs $Aj(V)$ sont mesurées pour différentes longueurs d'onde du filtre obtenues pour les valeurs de tensions correspondantes et les termes $x_i$ s'en déduisent facilement.

Le pouvoir calorifique H (P,T) du gaz s'écrit

$$\sum_{i = 1, ..., 5} x_i H_i$$

où $H_i$ représente le pouvoir calorifique du constituant i en Joules par mole.

Par conséquent, dès que les termes xi sont déterminés, le pouvoir calorifique H (P,T) est obtenu directement.

# EP 1 068 510 B1

**Revendications**

1. Procédé d'étalonnage en longueur d'onde d'un dispositif de filtrage (16) d'un rayonnement électromagnétique qui est inclus dans un appareil (10 ; 32) mesurant la transmission spectrale d'un milieu de propagation externe audit appareil et dans lequel milieu ledit rayonnement se propage, ledit dispositif de filtrage présentant une transmission spectrale accordable sur une gamme de longueurs d'onde dudit rayonnement en fonction de la valeur d'un paramètre physique, **caractérisé en ce que** ledit procédé consiste à:

   - sélectionner au moins une raie gazeuse d'absorption qui soit toujours présente à l'état naturel dans le milieu de propagation et dont la longueur d'onde correspondante soit incluse dans ladite gamme de longueurs d'onde d'accordabilité du dispositif de filtrage,
   - et étalonner le dispositif de filtrage par rapport à ladite au moins une raie gazeuse d'absorption qui sert de référence naturelle.

2. Procédé selon la revendication 1, consistant à sélectionner ladite au moins une raie gazeuse d'absorption qui soit, d'une part, d'une largeur spectrale inférieure ou égale à celle du dispositif de filtrage et, d'autre part, suffisamment intense pour ne pas être masquée par d'autres raies gazeuses.

3. Procédé selon la revendication 1 ou 2, consistant successivement à:

   - faire varier le paramètre physique appliqué au dispositif de filtrage (16) pour faire coïncider le maximum de la transmission spectrale dudit dispositif de filtrage avec la longueur d'onde de la raie gazeuse de référence,
   - en déduire le(s) coefficient(s) de la loi qui régit l'accordabilité du dispositif de filtrage (16) en longueur d'onde, l'allure générale de ladite loi étant préalablement connue,
   - déterminer à partir de cette loi d'autres valeurs du paramètre physique correspondant chacune à une longueur d'onde sur laquelle est accordée la transmission spectrale du dispositif de filtrage lors de son utilisation.

4. Procédé selon l'une des revendications 1 à 3, consistant à étalonner le dispositif de filtrage (16) lorsque l'appareil est installé sur son site d'utilisation.

5. Procédé selon l'une des revendications 1 à 4 selon lequel l'appareil comprend en outre:

   - au moins une source (12) du rayonnement électromagnétique et
   - un dispositif de détection (24) du rayonnement émis par la source, ladite source et ledit dispositif de détection (24) étant séparés par le milieu de propagation.

6. Procédé selon la revendication 5, consistant à interposer un volume de gaz dont on souhaite mesurer la transmission spectrale entre la source (12) et le dispositif de détection (24).

7. Procédé selon l'une des revendications 2 à 6, consistant à sélectionner sur ladite gamme de longueurs d'onde du rayonnement électromagnétique la raie gazeuse d'absorption qui présente la plus forte intensité par rapport aux autres raies gazeuses d'absorption.

8. Procédé selon l'une des revendications 1 à 7, consistant à sélectionner sur ladite gamme de longueurs d'onde du rayonnement électromagnétique deux raies gazeuses d'absorption.

9. Procédé selon l'une des revendications 1 à 8, selon lequel le rayonnement électromagnétique est du type infrarouge.

10. Procédé selon la revendication 9, selon lequel l'une des raies gazeuses d'absorption est celle du méthane à 1,666 microns.

11. Procédé selon la revendication 9 ou 10, selon lequel l'une des raies gazeuses d'absorption est celle du méthane à 1,791 microns.

12. Procédé selon l'une des revendications 1 à 11, consistant à appliquer au dispositif de filtrage comme paramètre physique un champ électrique sous la forme d'une tension électrique.

13. Procédé selon l'une des revendications 1 à 12, selon lequel le dispositif de filtrage est un interféromètre de Fabry-Perot (16).

14. Procédé selon la revendication 13, selon lequel l'interféromètre de Fabry-Perot (16) est un interféromètre court.

15. Procédé selon la revendication 13 ou 14, selon lequel l'interféromètre de Fabry-Perot (16) est un interféromètre micro-usiné.

16. Procédé selon l'une des revendications 1 à 15, selon lequel l'appareil est un analyseur de gaz (10 ; 32).

17. Procédé selon l'une des revendications 1 à 15, selon lequel l'appareil est Un appareil de mesure du pouvoir calorifique du gaz (10 ; 32).

18. Procédé selon l'une des revendications 1 à 15, selon lequel l'appareil est un capteur de gaz (10; 32).


**Patentansprüche**

1. Verfahren zur Wellenlängeneichung einer Vorrichtung (16) zur Filterung einer elektromagnetischen Strahlung, die in einem Gerät (10; 32) enthalten ist, das die spektrale Transmission eines Ausbreitungsmediums außerhalb des Geräts misst, wobei die Strahlung sich in diesem Medium ausbreitet, wobei die Filtervorrichtung eine auf einem Wellenlängenbereich der Strahlung in Abhängigkeit vom Wert eines physikalischen Parameters abstimmbare spektrale Transmission aufweist, **dadurch gekennzeichnet, dass** das Verfahren darin beruht,

   - wenigstens eine Gasabsorptionslinie auszuwählen, die im natürlichen Zustand in dem Ausbreitungsmedium immer vorhanden ist und deren entsprechende Wellenlänge in dem Wellenlängenbereich der Abstimmbarkeit der Filtervorrichtung enthalten ist,

   - und die Filtervorrichtung in Bezug auf diese wenigstens eine Gasabsorptionslinie zu eichen, die als natürliche Referenz dient.

2. Verfahren nach Anspruch 1, das darin beruht, die wenigstens eine Gasabsorptionslinie auszuwählen, die einerseits eine spektrale Breite kleiner oder gleichderjenigen der Filtervorrichtung aufweist und andererseits intensiv genug ist, um nicht von anderen Gaslinien verdeckt zu werden.

3. Verfahren nach Anspruch 1 oder 2, das darin beruht, nacheinander:

   - den an die Filtervorrichtung (16) angelegten physikalischen Parameter zu variieren, um das Maximum der spektralen Transmission der Filtervorrichtung mit der Wellenlänge der Referenz-Gaslinie in Übereinstimmung zu bringen,

   - davon den/die Koeffizienten der die Wellenlängenabstimmbarkeit der Filtervorrichtung bestimmenden Regel abzuleiten, wobei die allgemeine Gestalt der Regel zuvor bekannt ist,

   - anhand dieser Regel andere Werte des physikalischen Parameters zu bestimmen, die jeweils einer Wellenlänge entsprechen, auf die die spektrale Transmission der Filtervorrichtung bei ihrer Verwendung abgestimmt ist.

4. Vorrichtung nach einem der Ansprüche 1 bis 3, das darin beruht, die Filtervorrichtung (16) zu eichen, wenn das Gerät an seinem Anwendungsort installiert ist.

5. Verfahren nach einem der Ansprüche 1 bis 4, bei dem das Gerät ferner umfasst:

   - wenigstens eine Quelle (12) der elektromagnetischen Strahlung und

   - eine Erfassungsvorrichtung (24) zur Erfassung der von der Quelle ausgesendeten Strahlung, wobei die Quelle und die Erfassungsvorrichtung (24) durch das Ausbreitungsmedium getrennt sind.

**6.** Verfahren nach Anspruch 5, das darin beruht, ein Gasvolumen, dessen spektrale Transmission gemessen werden soll, zwischen der Quelle (12) und der Erfassungsvorrichtung (24) anzubringen.

**7.** Verfahren nach einem der Ansprüche 2 bis 6, das darin beruht, aus dem Wellenlängenbereich der elektromagnetischen Strahlung die Gasabsorptionslinie auszuwählen, die im Vergleich zu den anderen Gasabsorptionslinien die höchste Intensität aufweist.

**8.** Verfahren nach einem der Ansprüche 1 bis 7, das darin beruht, in dem Wellenlängenbereich der elektromagnetischen Strahlung zwei Gasabsorptionslinien auszuwählen.

**9.** Verfahren nach einem der Ansprüche 1 bis 8, bei dem die elektromagnetische Strahlung vom infraroten Typ ist.

**10.** Verfahren nach Anspruch 9, bei dem eine der Gasabsorptionslinien die des Methans bei 1,666 μm ist.

**11.** Verfahren nach Anspruch 9 oder 10, bei dem eine der Gasabsorptionslinien die des Methans bei 1,791 μm ist.

**12.** Verfahren nach einem der Ansprüche 1 bis 11, das darin beruht, an die Filtervorrichtung als physikalischen Parameter ein elektrisches Feld in Form einer elektrischen Spannung anzulegen.

**13.** Verfahren nach einem der Ansprüche 1 bis 12, bei dem die Filtervorrichtung ein Fabry-Perot-Interferometer (16) ist.

**14.** Verfahren nach Anspruch 13, bei dem das Fabry-Perot-Interferometer (16) ein kurzes Interferometer ist.

**15.** Verfahren nach Anspruch 13 oder 14, bei dem das Fabry-Perot-Interferometer (16) ein mikrobearbeitetes Interferometer ist.

**16.** Verfahren nach einem der Ansprüche 1 bis 15, bei dem die Vorrichtung ein Gasanalysator (10; 32) ist.

**17.** Verfahren nach einem der Ansprüche 1 bis 15, bei dem das Gerät ein Messgerät für den Brennwert des Gases (10; 32) ist.

**18.** Verfahren nach einem der Ansprüche 1 bis 15, bei dem das Gerät ein Gassensor (10; 32) ist.

**Claims**

**1.** A process for wavelength calibration of a device (16) for filtering an electromagnetic radiation which is included in an apparatus (10; 32) measuring the spectral transmission of a propagation medium outside of said apparatus and in which medium said radiation is propagated, said filtering device presenting a tunable spectral transmission on a range of wavelengths of said radiation according to the value of a physical parameter, **characterized in that** said process consists in:

- selecting at least one absorption gas line which is always present at the natural state in the propagation medium and the corresponding wavelength of which is included in said range of wavelengths of tunability of the filtering device,
- and calibrating the filtering device in relation to said at least one absorption gas line which is used as natural reference.

**2.** The process according to claim 1, consisting in selecting said at least one absorption gas line which is, on the one hand, of a spectral width lower or equal to that of the filtering device and, on the other hand, sufficiently strong not to be masked by other gas lines.

**3.** The process according to claim 1 or 2, consisting successively in:

- varying the physical parameter applied to the filtering device (16) to make the maximum of the spectral transmission of said filtering device coincide with the wavelength of the reference gas line,
- deriving the coefficient(s) from the law which governs the tunability of the filtering device (16) in wavelength, general pace of said law being known beforehand,

- determining from this law other values of the physical parameter each corresponding to a wavelength for which the spectral transmission of the filtering device during its use is tuned.

4. The process according to one of claims 1 to 3, consisting in calibrating the filtering device (16) when the apparatus is installed on its operation site.

5. The process according to one of claims 1 to 4, wherein the apparatus further includes:

   - at least one source (12) of the electromagnetic radiation and
   - a device (24) for detection the radiation emitted by the source, said source and said detection device (24) being separated by the propagation medium.

6. The process according to claim 5, consisting in interposing between the source (12) and the detection device (24) a volume of gas the spectral transmission of which one wishes to measure.

7. The process according to one of claims 2 to 6, consisting in selecting for said range of wavelengths of the electromagnetic radiation the absorption gas line which has the strongest intensity compared to the other absorption gas lines.

8. The process according to one of claims 1 to 7, consisting in selecting two absorption gas lines for said range of wavelengths of the electromagnetic radiation.

9. The process according to one of claims 1 to 8, wherein the electromagnetic radiation is of the infrared type.

10. The process according to the claim 9, wherein one of the absorption gas lines is the one of methane at 1.666 $\mu$m.

11. The process according to the claim 9 or 10, wherein one of the absorption gas lines is the one of methane at 1.791 $\mu$m.

12. The process according to one of claims 1 to 11, consisting in applying to the filtering device as a physical parameter an electric field in the form of an electric voltage.

13. The process according to one of claims 1 to 12, wherein the filtering device is a Fabry-Pérot interferometer (16).

14. The process according to the claim 13, wherein the Fabry-Pérot interferometer (16) is a short interferometer.

15. The process according to the claim 13 or 14, wherein the Fabry-Pérot interferometer (16) is a micromachined interferometer.

16. The process according to one of claims 1 to 15, wherein the apparatus is a gas analyzer (10, 32).

17. The process according to one of claims 1 to 15, wherein the apparatus is a measuring apparatus of the calorific value of the gas (10; 32).

18. The process according to one of claims 1 to 15, wherein the apparatus is a gas sensor (10; 32).

FIG. 1

FIG. 2

FIG. 3a          FIG. 3b

FIG. 4

FIG. 5

FIG. 6

FIG. 7